# EUROPEAN PATENT APPLICATION

(11) **EP 2 325 649 A1**
(43) Date of publication of application: **25.05.2011**
(21) Application number: 09306117.4
(22) Date of filing: 20.11.2009
(51) Int. Cl.: G01N 33/68

(54) **Dye-assisted laser desorption ionization (DALDI)**

(71) Applicant: Centre National de la Recherche Scientifique (CNRS), 75016 Paris (FR); Universite Des Sciences Et Technologies De Lille, 59650 Villeneuve D'ascq (FR)
(72) Inventor: Salzet, Michel, 59830 Bourghelles (FR); Fournier, Isabelle, 59830 Bourghelles (FR); Arafah, Karim, 59000 Lille (FR)
(74) Representative: Tetaz, Franck Claude Edouard

(57) **Abstract**

The present invention is related to dye assisted laser desorption ionisation mass spectrometry imaging (DALDI). This technology is particularly suitable for the detection and localisation of analytes such as lipids on biological tissue sections.

## Description

Lipids are a major component of cells and have varied roles in organisms. In a general manner, they play a key role in the structural composition of membranes but some like sphingoglycolipids are also mediators in different biological processes like protein transport, cellular growth regulation, cellular morphogenesis, neuronal plasticity, immune response regulation.

Lipidic diseases are due to dysfunction of the lipidic metabolism, characterized by accumulation of lipids of different types, especially cerebrosides, gangliosides or sphingomylelin in different organs. Accumulation of a particular type of lipid is often the consequence of dysfunction of one of specific enzyme (organics catalyser) that normally metabolizes this in the cell. In Gaucher disease, for example, an abnormal amount of cerebrosides is accumulated in liver, spleen, bone marrow and lymphatic ganglia. The deficient enzyme is the glucocerebrosidase. Excess of lipids stocked in the cells of Gaucher, stretched, typical of the disease, disrupts the functioning of the normal cell provoking two syndromes i.e. the acute cerebral form is most prevalent in infant, who is normal at birth but present rapidly a hypotonic development failure and severe neurologic symptoms, leading to the death, generally occurring the first year of the life; the chronicle form appears later. The characteristics are an increase of the spleen volume, anaemia and a coetaneous pigmentation with brown plaques.

Coronaries diseases like Fabry disease is due to case an intra-cellular accumulation of sphingolipids, particularly, globotriasylceramide and galactosylceramide, reflecting the preponderant role of lipids.

In this optic, Fujiwaki *et al.*¹⁻³ have demonstrated that the ratios of ceramide/monohesoxylceramide, ceramide/sphyngomyéline and glucosylceramide/sphyngomyeline in these two diseases as showing real differences.

Takeromi *et al*.⁴ have demonstrated the utility of MALDI for analysis of glycosphingolipids in human brain. These studies have been performed using the same technical preparation of lipids including extraction, centrifugation, and sodium carbonate treatment following lipids separation^{5,6} with sometimes a saponification treatment for quantification⁷ of sulfatides. Recently, direct analyses of the biological tissue by mass spectrometry have been developed⁸⁻¹⁰ and after a focus on proteins/peptides^{11,12}, lipids are now intensively investigated. Ishida *et al.* and later Rujoi *et al.* have used such technology for the localisation of phospholipids and/or sphinglomyelins in zooplankton, bacteria¹³⁻¹⁵ or in calf eyes¹⁶⁻¹⁸ In the same optic, imaging of phospholipids and cholesterol after direct analysis was firstly realized by SIMS-TOF in mice providing a map of these molecules in a slice¹⁹. Using MALDI, the first maps of lipids with mouse brains or liver by MALDI-MSI were performed very recently²⁰⁻²⁴. The major problem with lipids is both the sample preparation and the matrix. Lipids are moving during tissue preparation and are dissolved in the solvents used for matrix preparation. Thus, delocalization of lipids is frequent and is a problem in MALDI-MS imaging. Murphy's team²² used sublimation of 2,5-dihydroxybenzoic acid without the use of solvent that dissolves the lipid in the tissue. Whereas, Burnum *et al.* have used 2,5-dihydroxyacetophenone with aniline²⁵ or solvent-free matrix deposition²⁶.

As mentioned above, one of the major problems with lipids are their plasticity, high diffusion and high solubility. This makes it difficult to ensure that lipids are not diffusing during tissue preparation. This is also a difficulty for matrix solution application.

The present invention provides a novel technology to investigate lipidomic particularly for clinical studies. The present application describes a novel method for investigating these molecules by using specific dyes that have the faculty to absorb at the laser UV wavelength of MALDI-MS and that allow the detection of specific classes of lipids in positive and negative modes. Profiling and molecular images can be easily obtained without any lipid delocalization especially after fixation while avoiding difficulties due to matrix application by using classical dyes as MALDI matrices for lipids analysis. This technology has been named "DALDI" for Dye Assisted Laser Desorption Ionization.

Surprisingly, it is possible to use colorant agents or dyes sharing the property to absorb at UV-laser wavelength in order to desorb especially lipids from tissue sections. After fixation of lipids with chrome^{27,28} or with osmium tetroxide²⁹⁻³², coumarin dyes can be used directly without any additional MALDI matrix. Taking into account this ability for some dyes, specific colorants were identified that can be used with MALDI and which are specific to lipids. We stained tissue sections with either Sudan-Black B or Nile Blue A or Oil Red O, and performed molecular images without the need for any automatic-micro-spotting device and without any delocalization of lipids. In the methods of the present invention, fixation of lipids may be used to avoid their delocalization within the tissue sections during manipulations prior to MS analysis. Classical fixation is used and for dyes which are soluble in solvents such as alcohol an additional post-chromization step as a fixative for the lipids may be used.

Advantageously, tissue sections prepared for histological studies can be directly used to retrieve molecular information without any further treatment of the tissue sections. This method may be easily implemented in hospitals for pathologists all the more since no sprayer or spotter is needed to deposit the matrix onto the tissue sections. DALDI uses histochemical colorant dyes commonly used by the pathologist. DALDI provides histochemical staining and molecular diagnostic in the same slide simply by putting the stained slide into a MALDI instrument without any other treatment including matrix recall for histochemical staining. The feasibility of DALDI in clinical diagnosis is demonstrated herein for ovarian cancer. After fixation and staining, dyes such as Nile Blue A and Oil red O are very selective and allow the detection of very specific lipid markers in the tumor region. DALDI provides a powerful and useful tool for pathologists helping them perform a clear and quick diagnostic. Moreover, DALDI is clearly compatible with pathologist's processes and preserves the histological structure of the sample.

### SUMMARY OF THE INVENTION

The present invention relates to methods for the detection of an analyte in a biological tissue section comprising the following steps:
- Staining the biological tissue section with a dye absorbing at MALDI laser UV wavelength range,
- Analysing the biological tissue by Matrix Assisted Laser Desorption Mass spectrometry Imaging (MALDI-MSI) wherein said dye is directly used as the MALDI matrix.

Preferably, said dye is absorbing at laser UV wavelength between 250nm-380nm.

In preferred embodiments, the dye is selected from acidic dyes, basic dyes, diazo dyes, coumarin dyes, azo dyes and phenoxazone basic dyes.

More preferred, the dye is selected from Nile Blue A, Sudan Black and Oil Red O.

Preferably, the methods comprise fixation of the biological tissue section prior to the staining step.

Fixation of the biological tissue section may be carried out with a fixative solution comprising formalin and post-chromisation in a solution comprising K₂Cr₂O₇.

Fixation may further comprise fixation of the biological tissue section with a fixative solution comprising osmium tetroxyde (OsO₄).

The methods preferably provide for the detection of lipids. Preferably, the analyte is selected from triglycerides, sulfatides, diglycerides, spingomyeline, fatty acids, phospholipids, phosphatidylcholine, cholesteryl esters, triacylglycerols, lipoproteins, gangliosides, sphingenine and ceramides.

In preferred embodiments, the biological tissue section is analysed by Matrix Assisted Laser Desorption Mass spectrometry Imaging in positive ion and/or negative ion reflector mode.

The Matrix Assisted Laser Desorption Mass spectrometry Imaging (MALDI-MSI) is advantageously carried out with a MALDI-TOF mass spectrometer.

The invention also relates to methods for the *in vitro* clinical diagnosis on a biological tissue section comprising
- Staining the biological tissue section with a dye absorbing at MALDI laser UV wavelength range,
- Analysing the biological tissue by Matrix Assisted Laser Desorption Mass spectrometry Imaging (MALDI-MSI) wherein said dye is directly used as the MALDI matrix.

Another object of the invention is methods for anatomopathology diagnosis and Matrix Assisted Laser Desorption Mass spectrometry Imaging (MALDI-MSI) analysis on the same biological tissue section comprising
- Histological staining of the biological tissue section with a dye absorbing at MALDI laser UV wavelength range,
- Analysing the histological staining of the biological tissue section,
- Analysing the same biological tissue section by Matrix Assisted Laser Desorption Mass spectrometry Imaging (MALDI-MSI) wherein said dye is directly used as the MALDI matrix.

### DETAILED DESCRIPTION OF THE INVENTION

Mass spectrometry is an analytical tool used for measuring the molecular mass of an analyte. Mass spectrometers can be divided into three fundamental parts, namely the ionization source, the analyzer and the detector.

One of the ionization methods used for the majority of biochemical analyses is MALDI (matrix assisted laser desorption ionization). MALDI is based on bombardment of sample molecules with a laser light to bring about sample ionization. A MALDI matrix is usually required to protect the sample and to facilitate ionization and desorption. The analyte or sample is embedded in this matrix.

Any analyser may be used in the methods of the present invention. Time-of-flight mass spectrometry is a common technique largely described in the litterature. TOF-MS is a method of mass spectrometry in which ions are accelerated by an electric field. The velocity of the ion depends on the mass-to-charge ratio. The time that it subsequently takes for the particle to reach a detector at a known distance is measured. This time will depend on the mass-to-charge ratio of the ion. From this time and the known experimental parameters one can find the mass-to-charge ratio of the ion.

In the methods of the present invention, the type of mass spectrometer advantageously used with MALDI is the TOF (time-of-flight) mass spectrometer. However, any suitable mass spectrometer may be used in the methods of the present invention.

The present invention relates to MALDI mass spectrometry imaging (MALDI-MSI). MALDI-MSI links the universal detection capability of mass spectrometry with the positional information of molecular histology, generating mass spectra correlated to known locations within a biological tissue. In MALDI imaging, tissue sections are mounted onto a support and a suitable MALDI matrix is applied to the tissue. The support is inserted into a MALDI mass spectrometer which records the spatial distribution of different analytes. Suitable image processing software is used to import data from the mass spectrometer to allow visualization of analytes on the optical image of the tissue section.

A MALDI-MSI process comprises two steps: preparation of the sample including deposition of the matrix followed by ionization and desorption of the analyte by intense short pulses of laser light. The matrix provides for absorption of energy from the laser light to desorb the analyte and promotes ionization.

The term "matrix" refers to a material which generates matrix-embedded analyte molecules (i.e. proteins) that are successfully desorbed by laser irradiation and ionized from the solid phase. The matrix usually consists of molecules of low molecular weight to provide for vaporization but large enough not to evaporate during sample preparation or while standing in the spectrometer. Matrices also have a strong optical absorption in the UV range, so that they rapidly and efficiently absorb the laser irradiation. In classical MALDI, the matrix solution is mixed with the sample or applied onto the sample. The organic solvent allows hydrophobic molecules to dissolve into the solution, while the water allows for water-soluble (hydrophilic) molecules to do the same. Then, the solvents vaporize, leaving only the re-crystallized matrix, but now with protein molecules spread throughout the crystals. The matrix and the analyte are said to be co-crystallized in a MALDI spot.

The methods of the present invention further bring together the anatomopathological diagnosis and the mass spectrometry characterization. Dyes classically used in histochemical staining of biological tissue sections are used as matrices for molecular analysis by MALDI-MSI. Surprisingly, before and after histochemical staining, the methods of the present invention do not require deposition of any additional MALDI matrix onto the biologiocal tissue sections. The methods of the present invention therefore avoid the numerous difficulties encountered with matrix deposition onto tissue sections.

These methods can be used to localize and identify analytes particularly lipids throughout a tissue or organism under different experimental or therapeutic conditions.

The present invention relates to methods for the detection of an analyte in a biological tissue section comprising the following steps:
- Staining the biological tissue section with a dye absorbing at MALDI laser UV wavelength range,
- Analysing the biological tissue by Matrix Assisted Laser Desorption Mass spectrometry Imaging (MALDI-MSI) wherein said dye is directly used as the MALDI matrix.

The methods of the present invention are preferably *in vitro* methods.

The term "dye" refers to a colored substance that has an affinity for a substrate to which it is being applied. Preferably, the dyes used in the methods of the present invention are suitable for histochemical staining. More preferably, the dyes used in the methods of the present invention have an affinity for lipids or bind to lipids. In preferred embodiments, the dyes are lipid staining dyes.

Advantageously, the methods of the present invention do not require deposition of a any additional MALDI matrix onto the biological tissue section before or after tissue staining. The dye used in the histochemical staining step directly plays the role of the MALDI matrix. After staining, the biological tissue section may be directly analysed by MALDI-MSI without any additional step or any deposition of a specific MALDI matrix.

The dyes of the present invention are used as MALDI matrices. They are able to absorb energy in the UV wavelength of the MALDI laser providing desorption and ionisation of the analyte. Preferably, the dye is absorbing at laser UV wavelength range between 250 nm-430 nm, preferably between 250nm-380nm and more preferably between 320-360nm. More preferably, the dye absorbs at 335nm and/or 355 nm.

Any suitable dye may be used in the methods of the present invention; preferred dyes are acidic dyes, basic dyes, diazo dyes, coumarin dyes, azo dyes and phenoxazone basic dyes.

In preferred embodiments, the dye is selected from Nile Blue A, Sudan Black and Oil Red O.

The methods of the present invention provide for detection of an analyte in a biological tissue section. The term "detection" refers both to localization and identification of the analyte in the tissue section. Any analyte and more particulary any biological analyte may be detected with the methods of the present invention. In preferred embodiments, the analyte is a lipid. Lipids are a broad group of naturally-occurring molecules which includes fats, waxes, sterols, monoglycerides, diglycerides, phospholipids, etc. Lipids may be broadly defined as hydrophobic or amphiphilic small molecules.

The analyte may be detected, localized and/or identified in any biological tissue section of interest. The biological tissue section preferably has a thickness comprised between 5 and 20µm. Any tissue or organism may be analyzed in the methods of the present invention. The tissue section is prepared according to known methods. Thin sections/slices are typically obtained from frozen tissues and applied onto glass plates. The tissue sections are then usually dried in a dessicator.

To avoid delocalization of the analyte in the tissue section during staining it is advantageous to carry out fixation of the tissue section prior to staining.

Fixation may be carried out by any known fixative solution or any known fixation method.

For the detection of some analytes such as lipids it is advantageous to carry out fixation of the biological tissue section prior to the staining step.

Fixation may comprise treatment with formaldehyde such as formalin followed by post-chromisation in a solution comprising K₂Cr₂O₇. Preferably, fixation may be carried out by treatment with Baker's solution followed by treatment by post-chromisation in a solution comprising K₂Cr₂O₇ and formalin.

Fixation may also comprise treatment with a secondary fixative such as osmium tetroxyde. The secondary fixative may typically comprise osmium tetroxyde and formalin.

For some dyes that are not soluble in water and which are soluble in solvents such as alcohol it is advantageous to carry out additional fixation with a fixative solution comprising osmium tetroxyde and formalin.

Depending on the dye used as matrix different analytes may be detected and identified. Preferably, the methods of the present invention are appropriate for the detection of lipids. In the present invention, dyes known for histochemical staining of lipids can advantageously replace classical MALDI matrices for the analysis of lipids.

In preferred embodiments, the analyte is selected from triglycerides, sulfatides, diglycerides, spingomyeline, fatty acids, phospholipids, phosphatidylcholine, cholesteryl esters, triacylglycerols, lipoproteins, gangliosides, sphingenine and ceramides.

Sudan Black dye advantageously allows the detection of lipids such as triglycerides, sulfatides, diglycerides, sphingomyelin, phospholipids and fatty acids.

Nile Blue A dye provides for the detection of lipids such as diglycerides, sphingomyelin, phospholipids, triglycerides and sulfatides.

Oil RED O dye provides for the detection of lipids such as diglycerides, sphingomyelin, phospholipids, triglycerides, sulfatides and gangliosides.

The biological tissue section may be analysed by Matrix Assisted Laser Desorption Mass spectrometry Imaging in positive ion and/or negative ion reflector mode. Depending on the dye and the analyte detected, positive or negative modes are preferred.

In the methods of the present invention, the type of mass spectrometer advantageously used with MALDI is the TOP (time-of-flight) mass spectrometer. However, any suitable mass spectrometer may be used in the methods of the present invention.

Another object of the present invention is a method for the *in vitro* clinical diagnosis on a biological tissue section comprising
- Staining the biological tissue section with a dye absorbing at MALDI laser UV wavelength range,
- Analysing the biological tissue by Matrix Assisted Laser Desorption Mass spectrometry Imaging (MALDI-MSI) without further deposition of a MALDI matrix onto the biological tissue section after staining, the dye being used directly as the MALDI-matrix.

The present invention combines the histological staining of analytes such as lipids and their localisation/identification by MALDI-MSI.

The methods allow detection of lipids by MALDI MS over the range M/z 1000.

MALDI imaging can be performed on the same slide of tissue section than the histological diagnosis.

### FIGURES

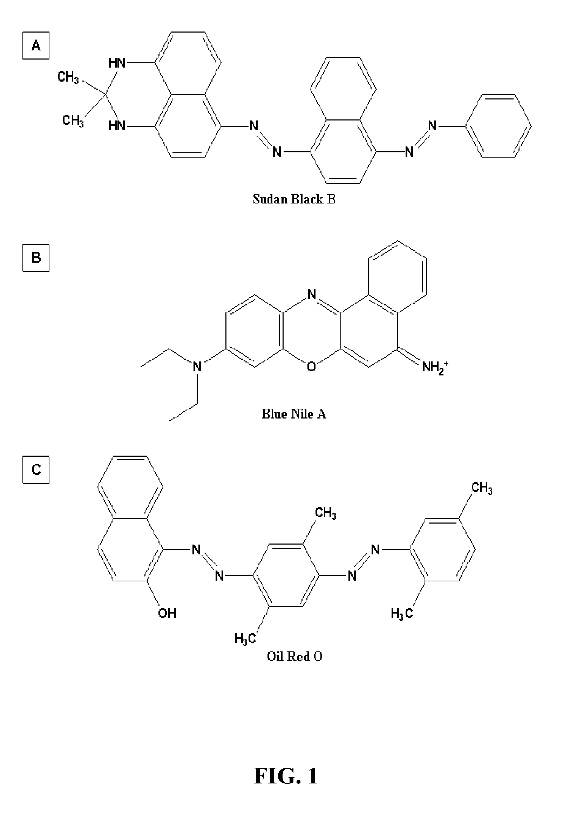

**Figure 1****:** Developed chemical formula of studied dyes **(A)** Sudan Black, **(B)** Nile Blue A and **(C)** Oil Red O.

### EXAMPLES

### Materials

2,6 dihydroxyacetophenone (2,6-DHAP), ethanol (EtOH), 2-amino-4-methyl-5-nitropyridine (2A4M5NP), water Chromasolv Plus for HPLC (H₂O), Nile Blue A, Oil red O, Black Sudan B, all certified by the Biological Stain Commission were purchased from Sigma-Aldrich (Saint Quentin Fallavier, France). 4% aqueous solution osmium tetroxyde OsO₄⁻ was purchased from Electron Microscopy Sciences (Hatfield, England). 330mm diameter filters were from Fisherbrand (Elancourt, France).

### Preparation of staining solutions

Nile Blue A was diluted in water (30 mg/ml). Oil red O and Sudan black B staining solutions were diluted in a 60% ethanol solution (30mg/mL). After stirring, all staining solutions were filtered.

### Rat brain cryosections and lipids fixation

Thin 10 µm tissue sections were obtained from frozen rat brain using a cryostat Leica CM1510S (Leica Microsystems, Nanterre, France) and applied onto ITO-coated conductive glass slides (Bruker Daltonics, Bremen, Germany). Rat brain slices were then dried in a dessicator for 10 minutes and subsequently fixed in the tetroxyde osmium solution prior staining with Sudan Black B for 15 minutes. Rat brain slices stained with Nile Blue A were fixed overnight in Baker's fixative solution and post-chromized for 2 hours prior lipid staining. Baker's fixative solution: 10 ml concentrated formalin, 10% concentration of CaCl2 and 90 mL of distilled water.

### Fixation protocol of rat brain lipids with osmium tetroxyde

A 1% osmium tetroxyde was prepared in water from the 4% mother stock solution. 50µL of the 1% running solution were deposited onto the rat brain slice and let for the fixation of lipids during 20 minutes under light cover.

### Protocol for global fixation of rat brain with Baker's fixative solution

A Baker's solution was prepared mixing 10mL of a 10% CaCl₂ solution to 10mL of concentrated formalin in 90mL of HPLC grade water. Slices were dipped overnight in the baker's solution for global tissue fixation and then, washed 3 times in HPLC grade water (3 bathes 5 minutes each). Slices were then dipped in a 50mL of a 5% K2Cr2O7 solution (m/v) and 7.5mL of formalin 4% all diluted in distilled water (HPLC grade). After 3 washes in HPLC grade water (3 bathes 5 minutes each), slices were dried for 15 minutes.

### Sample Preparation

### Rat brain cryosections

Thin 10 µm tissue sections were obtained from frozen rat brain using a cryostat Leica CM1510S (Leica Microsystems, Nanterre, France) and applied onto ITO-coated conductive glass slides (Bruker Daltonics, Bremen, Germany). Rat brain slices were then dehydrated in a dessicator for 10 minutes before deposition of the ionic matrix using Chip-1000 chemical inkjet printer (,Shimadzu, Kyoto, Japan).

### Ovarian cancer cryosections and fixation

Ovarian cancer biopsies were obtained, with informed consent and institutional review board approval (CCPPRBM Lille: CP 05/83), from patients undergoing any ovarian tumor resection at Hospital Jeanne de Flandre. Patient information was collected, including gender, age, treatment received before and after surgery, extent of surgery, current status (alive, alive with progressive disease, deceased, and cause of death), and survival from the time of original pathologic diagnosis. Samples were collected at the time of surgery, immediately frozen, and stored at -80°C until analysis.

Thin 12 µm were prepared from fresh ovarian cancer tissues using a cryostat Leica CM1510S (Leica Microsystems, Nanterre, France) and applied onto ITO-coated conductive glass slides (Bruker Daltonics, Bremen, Germany). The slices were first fixed in a Baker's fixative solution overnight and then postchromized for 2 hours for non solubilized hetephasic lipids (see protocol above). After drying into a dessicator for 15 minutes, slices were dipped into the Nile Blue A or the Oil Red O staining solution for 30 minutes or 15 minutes respectively. Histopathologic diagnoses were made by an anatomopathologist, blinded to the original clinical diagnosis, from subsequent H&E-stained sections.

### Matrices preparation

### Preparation 2,6 dihydroxyacetophenone (2,6 DHAP) matrix

2,6 DHAP matrix was used at 15mg/mL in EtOH/H₂O (1:1; v/v). The solution was stirred until total dissolution of the matrix.

### Preparation of the [2,6 DHAP/2A4M5NP] ionic matrix:

1 mole equivalent of 2,4,5 AMNP was added to 1 mole equivalent of 2,6 DHAP matrix prepared in EtOH/H₂O (1:1; v/v). Briefly, 15,1mg of the 2,4,5 AMNP (M= 153,14 g.mol⁻¹) was first dissolved in EtOH/H₂O (1:1; v/v). The preparation was then stirred 15 minutes and placed overnight at 60°C until complete dissolution. After cooling at room temperature, 15 mg of the 2,6 DHAP matrix (M= 152,15 g.mol⁻¹) were added to the solution and stirred briefly until dissolution of 2,6 DHAP. The ionic matrix can be used just after synthesis or kept several days at 60°C for further uses.

### Matrix deposition

20 nL of 2,6 DHAP in EtOH/H₂O (1:1; v/v) were deposited using the CHIP-1000. The spots were spaced by 250 µm center to center and 5 droplets of 100 pL were deposited at each spot per cycle, then 40 iterations were necessary to obtain the final volume. The solid ionic matrix DHAP/2A4M5NP was prepared and deposited using CHIP-1000 using the same parameters as for 2,6 DHAP. 2,6 DHAP was deposited onto the half-left part of the brain section and DHAP/2A4M5NP onto the half-right part.

### Mass Spectrometry Imaging of lipids

Molecular images were acquired using an UltraFlex II MALDI-TOF/TOF instrument (Bruker Daltonics, Bremen, Germany) equipped with a Smartbeam laser having a repetition rate up to 200 Hz and controlled by FlexControl 3.0 (Build 158) software (Bruker Daltonics, Bremen, Germany). Images were performed in negative reflector mode and MALDI MS spectra were acquired in the 200-1500 m/z range. A total of 500 spectra were acquired at each spot at a laser frequency of 100 Hz. The images were saved and reconstructed using FlexImaging 2.1 (Build 15) (Bruker Daltonics, Bremen, Germany).

### Statistical data analysis

Symbolic discriminant analysis (SDA) was used to analyze the lipid profiles. SDA allows determining discriminatory signals and builds functions using these signals that distinguish sample populations based on their classification. Peak lists from MALDI spectra obtained with *Flex analysis 2.4 and Flex control* 2.5 input into the analysis program were clustered according to similarity, using a presence/absence criterion. Statistical analyses were carried out using the ClinProTools 2.2 Software. For the statistical analyses, the mass spectra were internally recalibrated on common peaks (also known as spectral alignment) and normalized on the total ion count. An average spectrum created from all single spectra was used for a peak picking and to define integration ranges. These integration ranges were used to obtain the intensities or areas on the single spectra. The signal intensities were used for all calculations. For the principal component analysis and the hierarchical clustering, the individual peak intensities were standardized across the data set ¹². The PCA was carried out using Pareto scaling, which uses the square root of the standard deviation as a scaling factor to reduce the dominance of large-scale intensity changes in the matrix and other high-abundance ions, as these may mask variation in lower abundance ions during PCA. The overall outcome of PCA is greatly affected by the masking of the underlying relevant information by ions relating to matrix coating and other endogenous molecules. A plot of principal component 1 (PC1) versus principal component 2 (PC2) was chosen since these components resulted in the highest overall degree of separation of the spectra within the PCA scores plots. Under unsupervised PCA each spectrum is classed as an individual so the principal components are selected which account for the greatest separation of each of the individual spectra. At the top of each of the scores and loadings plots. The principal components that have been plotted are shown, with the percentage variance, or the percentage of the variance accounted for from each of the principal components.

### Ionic matrix for lipids

Lipids are largely and highly soluble in matrix solutions and can easily and quickly diffuse within tissues. Free solvent matrix deposition is a good alternative to minimize delocalization of lipids and generate very thin and homogenous crystallization polycrystalline layers of matrix^{26, 33}, although, fewer lipid species are generally observed using such approaches. Micro-spotting however is again a good compromise between analytical performances and analyte delocalization. In these cases, we have developed a novel ionic matrix which is easy to micro-spot with an automatic robot. This matrix is a combination of 2,6 dihydroxyacetophenone (2,6 DHAP) with 2-amino 4-methyl 5-nitropyridine (2A4M5NP). 2A4M5NP was previously used for reaction with HCCA, and HCCA/2A4MSNP has shown to present increased performances for tissue analysis³⁴. Half-left and half-right sides of the brain were respectively micro-spotted with 2,6 DHAP and 2,6 DHAP/2A4M5NP. Immediately after spotting of matrices, an optical scanning of the tissue section was performed to observe matrix crystallization of the 2,6 DHAP matrix and the ionic matrix 2,6 DHAP/2A4M5NP. This same tissue slices were then scanned after 12H under high vacuum inside the MALDI source of the instrument after having recorded molecular images of lipids with respectively 2,6 DHAP matrix and 2,6 DHAP/2A4M5NP ionic matrix. As observed from the pictures, 2,6 DHAP micro-spots have partially disappeared especially from the top and outside of the tissue section. This is due to matrix sublimation under vacuum conditions. In fact, 2,6 DHAP is known to present interesting properties for lipids analysis³⁵ from tissue sections but also to be very unstable under vacuum³⁵. By comparison, 2,6 DHAP/2A4M5NP matrix micro-spots were still present. Ionic matrices have proved to be extremely stables under vacuum conditions ³⁴ as exemplified here. Average mass spectra recorded after 12H under vacuum conditions show very low level of detection for lipids with 2,6 DHAP matrix whereas high intense signals are observed with DHAP/2A4M5NP ionic matrix. This is confirmed by the molecular images recorded with both matrices as shown for m/z 302 and m/z 408 ions. MALDI images obtained using 2,6 DHAP/2A4M5NP show a good correlation between the molecular images and the rat brain tissue structures whereas localization is rather unclear with 2,6 DHAP. These observations are confirmed on the other molecular images reconstructed from lipid signals ranging from m/z 300 to 1000. As an ionic matrix with 2A4M5NP, 2,6 DHAP is much more stable under vacuum conditions since ionic matrices do present a much higher sublimation temperature and the number lipid species detected with this matrix is higher than with 2,6 DHAP **(Table 1).**

**Table 1: Comparison of lipid species detected after application of 2,6 DHAP or 2,6 DHAP/2A4M5NP (in red).**

| **DHAP** **experimental** **mass (u.)** | **DHAP** **Intensity** **(a.u.)** | ***DHAP- 2A4M5NP*** **experimental** **mass (u.)** | **Intensity** **(a.u.)** | **Calculated** **mass (u.)** | **Identified** **species** | **Other** **identification** | **Calculated** **mass (u.)** |
|---|---|---|---|---|---|---|---|
| n.o. | | 263.17 | 975.24 | 263.16 | C16:4(OH) | | |
| n.o. | | 272.45 | 4371.35 | | | | |
| n.o. | | 287.55 | 2253.06 | 287.23 | C16:0(H₂0) | | |
| 302.07 | 117.4 | 301.96 | 9823.91 | | | | |
| 328.99 | 1140.29 | n.o. | | 329.26 | MG 16:0 | | |
| 341.86 | 116.59 | n.o. | | 341.31 | MG 18:1 | | |
| 404.11 | 123.51 | n.o. | | | | | |
| 408.99 | 9.06 | 409.14 | 1803 | 409.23 | PA 16:0 | | |
| n.o. | | 423.68 | 3505.68 | | | | |
| n.o. | | 317.95 | 2727.46 | | | | |
| n.o. | | 451.1 | 10848.78 | 451.28 | PA 19:0 | | |
| 531.51 | 1304.41 | n.o. | | 531.4 | DAG 30:4 | | |
| n.o. | | 602.39 | 1836.12 | 602.39 | PE 26:2 | | |
| 680.44 | 228.88 | n.o. | | 680.43 | PE 32:5 | | |
| n.o. | | 790.62 | 1140.48 | 790.56 | PS 36:0 | PE(18:0/22:6) | 790.54 |
| n.o. | | 835.24 | 901.05 | 835.53 | PI 34:1 | PG 41:6 | 835.55 |
| n.o. | | 863 | 269.66 | 862.61 | ST 40:1 | | |
| n.o. | | 879.06 | 819.16 | 878.06 | ST-OH 40:1 | PIP 32:5 | 879.40 |
| n.o. | | 886.01 | 1704.21 | 886.62 | ST 42:3 | | |
| 890.05 | 137.22 | 890.23 | 1444.68 | 890.6 | ST 42:1 | | |
| 907.1 | 195.78 | 907.59 | 2948.15 | 907.63 | ST-H₂0 42:1 | | |

| | | | | | | | |
|---|---|---|---|---|---|---|---|
| n.o. not observed | | | | | | | |

For all ions, a good correlation with the structures of the brain is observed using the ionic matrix. The ionic matrix combines both the analytical performances of 2,6 DHAP with stability under vacuum. It is also much more compatible with micro-spotting mode of matrix deposition. In fact, ionic matrices because of lower vapor pressure and slower crystallization have shown to be much better suited for micro-spotting devices. Thus, 2.6 DHAP/2A4M5NP is a new ionic matrix for lipids analysis with very interesting properties with respect to MALDI-MSI applications. ionic matrix fully suitable for MALDI-MSI studies of lipids, 2,6 dihydroxyacetophenone with 2-amino-4-methyl-5-nitropyridine (DHAP/2A4M5NP) provides for good extraction of lipids combined with a better stability under vacuum and simple deposit using automatic robot like CHIP 1000 (Shimadzu) or spay with Imageprep (Bruker Daltonics).

DHAP/2A4M5NP is greatly facilitating MS/MS structural studies in positive and negative modes and is easy to spot or spray automatically with known robots.

### Lipids fixation and Dye Assisted Laser Desorption /Ionization (DALDI) lipids MALDI-MSI

Fixed tissue sections were immerged in specific colorant solutions which are fluorescent hydrophobic probe. We used Nile blue A which is specific of phenoxazone basic dye and specific of neutral and acidic lipids as phospholipids like phosphatidylcholine as well. Acidic dyes like the Oil red O, a diazo dye specific for cholesteryl esters and neutral fats as triacylglycerols or the black Sudan, also a diazo dye that stains triglycerides and lipoproteins were assessed for imaging lipids with the MALDI technology. Nile blue A, Oil Red O and Sudan Black B present UV absorptions λ equal to 340 nm, 359 nm and 415 nm, which all are in the same λ than the MALDI UV laser absorption wavelength range.

Based on such properties, rat brain sections were treated with Baker and post-chromization and stained with colorant dyes. For Sudan Black section a step is added by a treatment with osmium tetroxide before coloration in order to unsure that no delocalization can occurs due to the presence of ethanol as vehicle for the dissolution of the dye.

Mass spectra and molecular images comparison between osmium tetroxyde fixed and stained with Sudan black or not, shown better lipids detection and structure preservation in fixed tissue. Comparison of molecular images for identical ions in both cases shows a very good localization in specific regions without any osmium tetroxyde-fixed tissue. Triglycerides, sulfatides, digyclerides, sphingomyeline and fatty acids are much better detected with Sudan Black B with fixation whereas phospholipids are better detected with Sudan Black without fixation **(Table 2).**

**Table 2: Comparison of lipid species detected after application of Black Sudan B with or without previous lipid fixation with osmium tetroxide.**

| **Experimental** **mass (u.)** | **Experimental** **intensity (a.u.)** | **Identified** **species** | **Calculated** **mass (u.)** | **Precision** **(u.)** **ΔM(exp-theo)** | **Other** **identification** | **Calculated** **mass (u.)** |
|---|---|---|---|---|---|---|
| **Black Sudan. Negative Mode. Without Fixative** | | | | | | |
| 249.20 | 509.80 | C16:3 | 249.19 | 0.01 | | |
| 255.65 | 175.58 | C16:0 | 255.23 | 0.42 | | |
| 282.85 | 124.09 | | | | | |
| 309.54 | 92.13 | C20:1 | 309.28 | 0.26 | | |
| 335.95 | 101.25 | C22:2 | 335.3 | 0.65 | | |
| 443.41 | 1354.61 | | | | | |
| 456.65 | 1094.44 | | | | | |
| 880.33 | 357.85 | PS(226/5:2 2/6) | 880.51 | -0.18 | | |
| 882.85 | 402.36 | PC(O-18:0/26:2) | 882.73 | 0.12 | PC(O-18:1/26:1) | 882.73 |
| 890.76 | 4224.96 | ST 42:1 | 890.63 | 0.13 | | |
| 904.55 | 109.09 | ST-OH 42:2 | 904.62 | -0.07 | PS 45:6 | 904.60 |
| 906.72 | 1556.30 | ST-OH | 906.62 | 0.10 | PS 45:5 | 906.62 |
| 1130.14 | 33.58 | GL 72:6 | 1130.01 | 0.13 | | |
| **Black Sudan. Negative Mode. With Os04 fixative** | | | | | | |
| 246.74 | 604.77 | | | | | |
| 255.24 | 136.65 | C16:0 | 255.23 | 0.01 | | |
| 265.54 | 247.25 | C16:3(OH) | **265.18** | 0.36 | C16:2(Ep) C16:2(Ke) | |
| 283.37 | 524.4 | C18:0 | 283.26 | 0.11 | | |
| 309.56 | 122.34 | C20:1 | 309.28 | 0.28 | | |
| 443.1 | 1138.72 | | | | | |
| 456.83 | 3194.84 | | | | | |
| 463.13 | 602.41 | SM 0:0 | 463.33 | -0.2 | | |
| 466 | | sphingani ne SM0:0 | 465.34 | 0.66 | | |
| 695.07 | 290.88 | GL 42:6 | 695.56 | -0.49 | | |
| 880.49 | 1648.57 | PS(22/5:2 2/6) | 880.51 | -0.02 | | |
| 883.68 | 350.10 | PG 44:3 | 883.64 | 0.04 | PI(38:5) PA(49:1) | 883.53 883.71 |
| 900.63 | 213.14 | ST-OH 42:2 | 900.60 | 0.03 | | |
| 906.69 | 4423.35 | ST-OH 42:1 | 906.62 | 0.07 | PS 45:5 | 906.62 |
| 921.72 | 207.69 | PA 52:3 | 921.73 | -0.01 | PC 46:4 | 921.71 |
| 1020.03 | 256.33 | PI 47:0 | 1019.75 | 0.28 | PI(44:4) | 149.61 |
| 1050.91 | 315.44 | PI(24:4/2 6:2) | 1049.71 | 1.2 | | |

In contrary, phospholipids are better detected with Sudan Black without fixation **(Table 2)** but the spatial localization of these compounds is completely lost, washed by the ethanol/water vehicle once the slide is diped into the staining solution. In the case of Nile Blue A, after fixation with the Baker fixative solution and post-chromization of lipids for their insolubilization, negative and positive modes can be used. In positive mode, diglycerides, sphingomyelin, phospholipids, triglycerides and sulfatides are localized with respect to the rat brain anatomy **(Table 3).**

**Table 3: Comparison of lipid species detected in positive and negative mode after application of Nile blue A**

| **Experimental** **mass (u.)** | **Intensity** **(a.u.)** | **Calculated** **mass (u.)** | **Identification** **[M-H]- species** | **Precision** **(u.)** **ΔM(exp-theo)** | **Other** **identification** | **Calculated** **mass (u.)** |
|---|---|---|---|---|---|---|
| Nile Blue A, Negative mode | | | | | | |
| 197.49 | 3902.22 | | | | | |
| 288.51 | 368.11 | | | | | |
| 506.31 | 41.16 | 506.36 | PC(O-18:1/0:0) | -0.05 | | |
| 524.2 | 44.53 | 524.27 | PE 22:6 | -0.07 | PE(18:0) | 524.29 |
| 631.28 | 59.41 | | | | | |
| 720.42 | 48.13 | 720.46 | PE 35:6 | -0.04 | PC(32:6) | 740.46 |
| 739.46 | 55.23 | 739.58 | GL 44:5 | -0.12 | GL(45:5) | 739.62 |
| 782.59 | 38.76 | 782.65 | CerP(d18:1/24:0) | -0.06 | | |
| 811.58 | 78.54 | 811.67 | SM 24:1 | -0.09 | PA(44:2) | 811.62 |
| 882.47 | 102.15 | 882.52 | PS 44:10 | -0.05 | | |
| 892.7 | 124.26 | 892.63 | ST 42:0 | 0.07 | | |
| 907.1 | 254.58 | | ST-OH? | | | |
| 923.34 | 121.53 | 923.47 | PIP 35:4 | -0.13 | | |
| 1039.91 | 67.22 | 1040 | GL 66:2 | -0.09 | | |

| Nile Blue A, Positive mode | | | | | | |
|---|---|---|---|---|---|---|
| 184.16 | 9232.86 | 184 | Pcho | 0.16 | | |
| 274.12 | 2686.91 | | | | | |
| 281.24 | 2627.66 | 281.24 | C18:2 | 0 | | |
| 291.07 | n.d. | | C14 species | | | |
| 303.16 | 2509.41 | 303.25 | MG(14:0) | -0.09 | | |
| 332.36 | 782.84 | | | | | |
| 405.21 | 19561.98 | 405.29 | MG 22:5 | -0.08 | | |
| 422.22 | 790.54 | 422.26 | CerP 2:0 | -0.04 | | |
| 441.32 | 362.78 | 441.39 | DG 24:1 | -0.07 | | |
| 459.26 | 11859.23 | 459.25 | PA 20:4 | 0.01 | | |
| 474.29 | 1813.26 | 474.26 | PE 18:4 | 0.03 | | |
| 502.3 | 411.57 | 502.29 | PE 18:4 | 0.01 | | |
| 583.38 | 671.51 | 583.39 | PG 23:0 | -0.01 | PG 22:0 | 583.36 |
| 613.32 | 3002.18 | 613.38 | PA 30:4 | -0.06 | | |
| 618.45 | 4292.47 | 618.48 | Sphinganine CerP 16:0 | -0.03 | | |
| 634.5 | 3627.95 | 634.48 | PC 26:1/PE 29:1 | 0.02 | sphinganine Cer20:0 (K) | 634.55 |
| 722.56 | 2428.58 | 722.54 | Sphingosine CerP 24:4 | 0.02 | PS 32:0 | 722.53 |
| 740.43 | 452.38 | 740.45 | PS 33:5 | -0.02 | | |
| 760.42 | 534.19 | 760.41 | | 0.01 | PS 33:6 (Na) | 760.41 |
| 789.36 | 656.52 | 789.39 | PIP 26:2 | -0.03 | | |
| 861.48 | 213.14 | 861.49 | PIP(O-18:O-13) | -0.01 | | |
| 879.67 | 1313.8 | 879.68 | PA 49:4 | -0.01 | | |
| 915.47 | 1072.62 | 915.49 | PIP 34:2 | -0.02 | | |
| 1072.17 | 295.05 | 1072.06 | GL 68:0 | 0.11 | | |

The last dye, Oil red O allows the detection of all these lipids but also gangliosides. These data reflect real complementary methods for lipids localization due to the possibility to get access to negative and positive modes. Molecular images reinforce this assumption. Experiments were also performed using Oil Red O staining in combination with fixation process. Similar observations can be drawn from these experiments. Better correlation between tissue structures is again obtained for fixed samples. However, with Oil Red O, different classes of lipids are better analyzed like PE 42:11, PE 44:11, ST-OH 40: 0, ST-OH 42:1, ST-44:1. These experiments highlight the interest of performing lipids fixation to avoid diffusion of such species inside tissue section. This also shows that classical dyes for lipids can advantageously replace classical MALDI matrices for lipids analysis. Thus, it is possible to prepare a tissue section for histological studies of lipids and use the same tissue section directly for MALDI-MSI without the need for applying a further matrix. This provides an interesting alternative avoiding difficulties due to matrix application. Moreover, a good quality of spectra at least equal to what is obtained with 2,6 DHAP is recorded using these dyes as matrices. The results obtained show that it is possible to use fixed and stained tissue sections prepared for histology analysis for MALDI-MSI without any further treatment of the tissue section. This technology has been is called "Dye Assisted Laser Desorption Ionization (DALDI)".

### Application to pathology: Ovary cancer

In order to investigate, these developments on pathology, we tested Nile Blue A and Oil Red O on serous ovarian carcinoma after osmium fixation **(Table 4).** Principal component analyses were performed on the biopsies allowing to define two regions have been detected. As observed from **Table 4,** more lipid species are detected with the Nile blue A than with the Oil Red O for ovarian cancer biopsies.

**Table 4: Comparison of lipid species detected in human cancer biopsies after application of either Oil red O or Nile Blue A.**

| **Experimetal** **mass (u.)** | **Intensit y** **(a.u.)** | **Calculated** **mass (u.)** | **Identification** **[M-H]- species** | **Precision (u.)** **ΔM(exp-theo)** | **Other** **identification** | **Calculated** **mass (u.)** |
|---|---|---|---|---|---|---|
| Ovarian Cancer Oil Red O | | | | | | |
| 183.93 | 1251.67 | | | | | |
| 200.01 | 4630.16 | 200.1 | C10:1(H2O) | -0.09 | | |
| 284.06 | 3601.5 | | | | | |
| 285.12 | 2085.36 | 285.13 | C14:3 | -0.01 | | |
| 300.14 | 979.61 | 300.29 | SPhinganine Cer0:0 | -0.15 | | |
| 305.22 | 102.43 | 305.24 | eicosatrienoic acid C20:3 | -0.02 | | |
| 407.35 | 316.87 | 407.35 | hexacosadieno ic acid C22:6 | 0 | | |
| 421.23 | 1665.04 | 421.23 | phosphatidic acid PA 17:1 | 0 | | |
| 422.23 | 950.02 | 422.26 | sphinganine CerP2:0 | -0.03 | PE(14:1/0:0) | |
| 452.24 | 332.77 | 452.26 | PE 16:0 | -0.02 | | |
| 536.38 | 64.74 | 536.37 | PC 19:0 | 0.01 | | |
| 552.32 | 14 | 552.33 | PS 20:0 | -0.01 | PE 22:0 | |
| 553.37 | 73.64 | 553.36 | phosphoglyceri de PG 21:0 | 0.01 | C30:5 | |
| 604.4 | 176.43 | 604.39 | PE 26:1 | 0.01 | | |
| 620.48 | 40.23 | 620.46 | PE(O-16:0/12:0) | 0.02 | PE(O-18:0/10:0). PC(25:0/0:0) | |
| 785.66 | 35.54 | 785.65 | sphingomyelin 22:0 | 0.01 | PA(O-18:1/25:0). GL 47:3 | |
| 886.64 | 14.83 | 886.63 | PC 44:7 | 0.01 | | |

| Ovarian Cancer, Nile Blue A | | | | | | |
|---|---|---|---|---|---|---|
| 163 | 7626.05 | 163.07 | C10:4 | -0.07 | | |
| 196.1 | 2648.07 | | | | | |
| 200.15 | 2001.34 | | | | | |
| 255.63 | 68.22 | 255.23 | C16:0 | 0.4 | | |
| 282.27 | 154.24 | | C18:0 | | | |
| 283.63 | 109.29 | | DiCH3C16:0 | | | |
| 285.12 | 471.76 | 285.15 | C18:6(Ep) | -0.03 | C18:6(Ke) | |
| 291.1 | 791.68 | 291.19 | C18:4(OH) | -0.09 | C18:3(OH)-cyclo | |
| 297.43 | 345.03 | 297.25 | C18:1(OH) | 0.18 | | |
| 300.45 | 756.37 | 300.29 | Sphinganine Cer0:0 | 0.16 | | |
| 303.29 | 152.44 | 303.24 | C20:4 | 0.05 | | |
| 326.31 | 494.43 | | | | | |
| 369.14 | 342.95 | | | | | |
| 384.95 | 429.95 | | | | | |
| 405.9 | 578.21 | | | | | |
| 459.12 | 200.28 | 459.25 | PA 20:3 | -0.13 | | |
| 474.1 | 175.31 | 474.26 | PE 18:3 | -0.16 | | |
| 579.37 | 35.15 | 579.49 | TG 33:1 | -0.12 | | |
| 600.51 | 109.71 | 600.33 | PS 24:4 | 0.18 | SphingosineC er18:0(CI-) Sphinganine Cer18:1(CI-) | 600.51 |
| 614.5 | 128.67 | 614.46 | sphingosineHe xCer10:0 | 0.04 | | |
| 721.73 | 127.61 | 721.7 | TG 44:0 | 0.03 | DAG 33:0 | 721.54 |
| 740.75 | 449.28 | 740.45 | PC 33:4 | 0.3 | | |
| 881.18 | 110.47 | | TG? | | | |
| 896.1 | 31.25 | | TG? | | | |
| 935.26 | 34.51 | 935.46 | PIP 36:5 | -0.2 | | |
| 978.46 | 31.96 | 979.53 | PIP 39:4 | -1.07 | | |

However, considering PCA analyses, some specific ions (421.84, 422.85, 407.35) have been detected in the tumor region with Oil red O. These ions may correspond to the hexacosadienoic acid C22:6 (407.35), the phosphatidic acid PA 17:1 (421.23) and the sphinganine CerP2:0 (422.26). With Nile Blue A, the results are successful as well. Some specific ions have been detected in the tumor region after PCA analyses like the 255.63 (palmitic acid), the 303.29 (arachidonoic acid), the 283.63 (hexadecoid acid), the 282.25 (C18:0) or the 369.14 which is unknown **(Table 4).** These data are in line with previous studies having shown that fatty acid compositions of ceramide and sphingomyelin isolated from tissues are significantly different: the predominant acids are oleic (18:1) in ceramide and palmitic (16:0) in sphingomyelin, which contains much more other saturated acids. These differences are characteristic of both normal and tumor tissues. Sphingoid base compositions of ceramide and sphingomyelin in normal tissue are identical: the major component is sphingenine (over 96%), while ceramides from tumors contain, in addition to sphingenine, a significant amount of sphinganine. In sphingomyelins from tumours, sphinganine content is significantly lower than in ceramides and depends on the type of tumour ^{36, 37} Considering our data, applying Oil red O and Nile blue A as coloring agents to study ovarian carcinoma not only succeed in detecting lipids from this tissue by MALDI MSI but put also forward the capability of DALDI MSI to highlight potential biomarkers like sphingenine in combination with PCA analyses.

Moreover, these lipid-related dyes provide the possibility to detect preferentially some groups of lipids over others taking into account the chemical properties of basic dyes such as Nile Blue A or acidic dyes such as Sudan Black B or Oil Red O in addition with the working solvent. In comparison, the widely used MALDI matrices giving poor drawbacks in low m/z for the investigation of lipidome in mass spectrometry are acidic. As seen herein, the Nile Blue A dissolved in water is more lipophilic with neutral and acidic lipids taking affinity with phospholipids and sulfatides in negative mode whereas much more lipids are detected in positive mode according to the basic property of this dye (Table 3). In this latter mode of detection, the Nile Blue A covers fatty acids, glycerides, sphingo and phospholipids. The use of this dye takes advantage in its better partition into hydrophobic tails of lipids instead of water when the slices are deeped into this staining solution. As a consequence of the water vehicle, a minimized délocalization can easily been corrected just by fixation of the biological sample slice in Baker's fixative following by a post-chromization step.

### NON-PATENT LITTERATURE CITED IN THE DESCRIPTION

1. Fujiwaki, T.; Tasaka, M.; Yamaguchi, S., Quantitative evaluation of sphingomyelin and glucosylceramide using matrix-assisted laser desorption ionization time-of-flight mass spectrometry with sphingosylphosphorylcholine as an internal standard. Practical application to tissues from patients with Niemann-Pick disease types A and C, and Gaucher disease. J Chromatogr B Analyt Technol Biomed Life Sci 2008, 870, (2), 170-6.
2. Fujiwaki, T.; Yamaguchi, S.; Tasaka, M.; Sakura, N.; Taketomi, T., Application of delayed extraction-matrix-assisted laser desorption ionization time-of-flight mass spectrometry for analysis of sphingolipids in pericardial fluid, peritoneal fluid and serum from Gaucher disease patients. J Chromatogr B Analyt Technol Biomed Life Sci 2002, 776, (1), 115-23.
3. Fujiwaki, T.; Yamaguchi, S.; Tasaka, M.; Takayanagi, M.; Isobe, M.; Taketomi, T., Evaluation of sphingolipids in vitreous bodies from a patient with Gaucher disease, using delayed extraction matrix-assisted laser desorption ionization time-of-flight mass spectrometry. J Chromatogr B Analyt Technol Biomed Life Sci 2004, 806, (1), 47-51.
4. Taketomi, T.; Hara, A.; Uemura, K.; Sugiyama, E., Rapid method of preparation of lysoglycosphingolipids and their confirmation by delayed extraction matrix-assisted laser desorption ionization time-of-flight mass spectrometry. J Biochem 1996, 120, (3), 573-9.
5. Bligh, E. G.; Dyer, W. J., A rapid method of total lipid extraction and purification. Can JBiochem Physiol 1959, 37, (8), 911-7.
6. Lebaron, F. N.; Folch, J., The effect of pH and salt concentration on aqueous extraction of brain proteins and lipoproteins. J Neurochem 1959, 4, (1), 1-8.
7. Calvert, G. D.; Blight, L.; Illman, R. J.; Topping, D. L.; Potter, J. D., A trial of the effects of soya-bean flour and soya-bean saponins on plasma lipids, faecal bile acids and neutral sterols in hypercholesterolaemic men. Br J Nutr 1981, 45, (2), 277-81.
8. Caprioli, R. M.; Farmer, T. B.; Gile, J., Molecular imaging of biological samples: localization of peptides and proteins using MALDI-TOF MS. Anal Chem 1997, 69, (23), 4751-60.
9. Fournier, I.; Day, R.; Salzet, M., Direct analysis of neuropeptides by in situ MALDI-TOF mass spectrometry in the rat brain. Neuro Endocrinol Lett 2003, 24, (1-2), 9-14.
10. Jimenez, C. R.; Li, K. W.; Dreisewerd, K.; Spijker, S.; Kingston, R.; Bateman, R. H.; Burlingame, A. L.; Smit, A. B.; van Minnen, J.; Geraerts, W. P., Direct mass spectrometric peptide profiling and sequencing of single neurons reveals differential peptide patterns in a small neuronal network. Biochemistry 1998, 37, (7), 2070-6.
11. Caprioli, R. M., Deciphering protein molecular signatures in cancer tissues to aid in diagnosis, prognosis, and therapy. Cancer Res 2005, 65, (23), 10642-5.
12. Franck, J.; Arafah, K.; Elayed, M.; Bonnel, D.; Vergara, D.; Jacquet, A.; Vinatier, D.; Wisztorski, M.; Day, R.; Fournier, I.; Salzet, M., MALDI IMAGING: State of the art technology in clinical proteomics. Mol Cell Proteomics 2009**.**
13. Ishida, Y.; Kitagawa, K.; Nakayama, A.; Ohtani, H., On-probe sample pretreatment for direct analysis of lipids in gram-positive bacterial cells by matrix-assisted laser desorption ionization mass spectrometry. Appl Environ Microbiol 2005, 71, (11), 7539-41.
14. Ishida, Y.; Madonna, A. J.; Rees, J. C.; Meetani, M. A.; Voorhees, K. J., Rapid analysis of intact phospholipids from whole bacterial cells by matrix-assisted laser desorption/ionization mass spectrometry combined with on-probe sample pretreatment. Rapid Commun Mass Spectrom 2002, 16, (19), 1877-82.
15. Ishida, Y.; Nakanishi, O.; Hirao, S.; Tsuge, S.; Urabe, J.; Sekino, T.; Nakanishi, M.; Kimoto, T.; Ohtani, H., Direct analysis of lipids in single zooplankter individuals by matrix-assisted laser desorption/ionization mass spectrometry. Anal Chem 2003, 75, (17), 4514-8.
16. Rujoi, M.; Estrada, R.; Yappert, M. C., In situ MALDI-TOF MS regional analysis of neutral phospholipids in lens tissue. Anal Chem 2004, 76, (6), 1657-63.
17. Rujoi, M.; Jin, J.; Borchman, D.; Tang, D.; Yappert, M. C., Isolation and lipid characterization of cholesterol-enriched fractions in cortical and nuclear human lens fibers. Invest Ophthalmol Vis Sci 2003, 44, (4), 1634-42.
18. Yappert, M. C.; Rujoi, M.; Borchman, D.; Vorobyov, I.; Estrada, R., Glycero-versus sphingo-phospholipids: correlations with human and non-human mammalian lens growth. Exp Eye Res 2003, 76, (6), 725-34.
19. Touboul, D.; Halgand, F.; Brunelle, A.; Kersting, R.; Tallarek, E.; Hagenhoff, B.; Laprevote, O., Tissue molecular ion imaging by gold cluster ion bombardment. Anal Chem 2004, 76, (6), 1550-9.
20. Astigarraga, E.; Barreda-Gomez, G.; Lombardero, L.; Fresnedo, O.; Castano, F.; Giralt, M. T.; Ochoa, B.; Rodriguez-Puertas, R.; Fernandez, J. A., Profiling and imaging of lipids on brain and liver tissue by matrix-assisted laser desorption/ ionization mass spectrometry using 2-mercaptobenzothiazole as a matrix. Anal Chem 2008, 80, (23), 9105-14.
21. Mikawa, S.; Suzuki, M.; Fujimoto, C.; Sato, K., Imaging of phosphatidylcholines in the adult rat brain using MALDI-TOF MS. Neurosci Lett 2009, 451, (1), 45-9.
22. Murphy, R. C.; Hankin, J. A.; Barkley, R. M., Imaging of lipid species by MALDI mass spectrometry. J Lipid Res 2009, 50 Suppl, S317-22.
23. Sugiura, Y.; Konishi, Y.; Zaima, N.; Kajihara, S.; Nakanishi, H.; Taguchi, R.; Setou, M., Visualization of the cell-selective distribution of PUFA-containing phosphatidylcho lines in mouse brain by imaging mass spectrometry. J Lipid Res 2009**.**
24. Sugiura, Y.; Shimma, S.; Konishi, Y.; Yamada, M. K.; Setou, M., Imaging mass spectrometry technology and application on ganglioside study; visualization of age-dependent accumulation of C20-ganglioside molecular species in the mouse hippocampus. PLoS One 2008, 3, (9), e3232.
25. Burnum, K. E.; Cornett, D. S.; Puolitaival, S. M.; Milne, S. B.; Myers, D. S.; Tranguch, S.; Brown, H. A.; Dey, S. K.; Caprioli, R. M., Spatial and temporal alterations of phospholipids determined by mass spectrometry during mouse embryo implantation. J Lipid Res 2009**.**
26. Puolitaival, S. M.; Burnum, K. E.; Cornett, D. S.; Caprioli, R. M., Solvent-free matrix dry-coating for MALDI imaging of phospholipids. J Am Soc Mass Spectrom 2008, 19, (6), 882-6.
27. Purbach, B.; Hills, B. A.; Wroblewski, B. M., Surface-active phospholipid in total hip arthroplasty. Clin Orthop Relat Res 2002, (396), 115-8.
28. Kotelnikov, V. M.; Litinskaya, L. L., Comparative studies of Feulgen hydrolysis for DNA. I. Influence of different fixatives and polyethylene glycols. Histochemistry 1981, 71, (1), 145-53.
29. Collin, R.; Griffith, W. P.; Phillips, F. L.; Skapski, A. C., Staining and fixation of unsaturated membrane lipids by osmium tetroxide. Crystal structure of a model osmium(VI) di-ester. Biochim Biophys Acta 1974, 354, (1), 152-4.
30. Collin, R.; Griffith, W. P.; Phillips, F. L.; Skapski, A. C., Staining and fixation of unsaturated membrane lipids by osmium tetroxide: crystal structure of a model osmium(VI) intermediate. Biochim Biophys Acta 1973, 320, (3), 745-7.
31. Litman, R. B.; Barmett, R. J., The mechanism of the fixation of tissue components by osmium tetroxide via hydrogen bonding. J Ultrastruct Res 1972, 38, (1), 63-86.
32. Riemersma, J. C., Osmium tetroxide fixation of lipids for electron microscopy. A possible reaction mechanism. Biochim Biophys Acta 1968, 152, (4), 718-27.
33. Hankin, J. A.; Barkley, R. M.; Murphy, R. C., Sublimation as a Method of Matrix Application for Mass Spectrometric Imaging. J Am Soc Mass Spectrom 2007**.**
34. Lemaire, R.; Tabet, J. C.; Ducoroy, P.; Hendra, J. B.; Salzet, M.; Fournier, I., Solid ionic matrixes for direct tissue analysis and MALDI imaging. Anal Chem 2006, 78, (3), 809-19.
35. Jackson, S. N.; Ugarov, M.; Post, J. D.; Egan, T.; Langlais, D.; Schultz, J. A.; Woods, A. S., A study of phospholipids by ion mobility TOFMS. J Am Soc Mass Spectrom 2008, 19, (11), 1655-62.
36. Rylova, S. N.; Somova, O. G.; Dyatlovitskaya, E. V., Comparative investigation of sphingoid bases and fatty acids in ceramides and sphingomyelins from human ovarian malignant tumors and normal ovary. Biochemistry (Mosc) 1998, 63, (9), 1057-60.
37. Dyatlovitskaya, E. V.; Andreasyan, G. O.; Malykh Ya, N.; Rylova, S. N.; Somova, O. G., Ganglioside shedding and changes in ceramide biosynthesis in human ovarian tumors. Biochemistry (Mosc) 1997, 62, (5), 557-61.

## Claims

1. Method for the detection of an analyte in a biological tissue section comprising the following steps :
a) Staining the biological tissue section with a dye absorbing at MALDI laser UV wavelength range,
b) Analysing the biological tissue by Matrix Assisted Laser Desorption Mass spectrometry Imaging (MALDI-MSI) wherein said dye is directly used as the MALDI matrix.

2. Method for the detection of an analyte in a biological tissue section according to claim 1 wherein the dye is absorbing at UV wavelength between 250nm-380nm.

3. Method for the detection of an analyte in a biological tissue section according to anyone of claims 1-2 wherein the dye is selected from acidic dyes, basic dyes, diazo dyes, coumarin dyes, azo dyes and phenoxazone basic dyes.

4. Method for the detection of an analyte in a biological tissue section according to anyone of claims 1-3 wherein the dye is selected from Nile Blue A, Sudan Black and Oil Red O.

5. Method for the detection of an analyte in a biological tissue section according to anyone of claims 1-4 comprising fixation of the biological tissue section prior to the staining step.

6. Method for the detection of an analyte in a biological tissue section according to claim 5 comprising fixation of the biological tissue section with a fixative solution comprising formalin and post-chromisation in a solution comprising K₂Cr₂O₇.

7. Method for the detection of an analyte in a biological tissue section according to claim 6 comprising secondary fixation of the biological tissue section with a fixative solution comprising osmium tetroxyde (OsO₄).

8. Method for the detection of an analyte in a biological tissue section according to anyone of claims 1-7 wherein the analyte is a lipid.

9. Method for the detection of an analyte in a biological tissue section according to anyone of claims 1-8 wherein the analyte is selected from triglycerides, sulfatides, diglycerides, spingomyeline, fatty acids, phospholipids, phosphatidylcholine, cholesteryl esters, triacylglycerols, lipoproteins, gangliosides, sphingenine and ceramides.

10. Method for the detection of an analyte in a biological tissue section according to anyone of claims 1-9 wherein the biological tissue section is analysed by Matrix Assisted Laser Desorption Mass spectrometry Imaging in positive ion and/or negative ion reflector mode.

11. Method for the detection of an analyte in a biological tissue section according to anyone of claims 1-10 wherein the Matrix Assisted Laser Desorption Mass spectrometry Imaging (MALDI-MSI) is carried out with a MALDI-TOF mass spectrometer.

12. Method for the *in vitro* clinical diagnosis on a biological tissue section comprising
a) Staining the biological tissue section with a dye absorbing at MALDI laser UV wavelength range,
b) Analysing the biological tissue by Matrix Assisted Laser Desorption Mass spectrometry Imaging (MALDI-MSI) wherein said dye is directly used as the MALDI matrix.

13. Method for anatomopathology diagnosis and Matrix Assisted Laser Desorption Mass spectrometry Imaging (MALDI-MSI) analysis on the same biological tissue section comprising
a) Histological staining of the biological tissue section with a dye absorbing at MALDI laser UV wavelength range,
b) Analysing the histological staining of the biological tissue section,
c) Analysing the same biological tissue section by Matrix Assisted Laser Desorption Mass spectrometry Imaging (MALDI-MSI) wherein said dye is directly used as the MALDI matrix.
